## Europäisches Patentamt
## European Patent Office
## Office européen des brevets

(19)

(11) Veröffentlichungsnummer: **0 166 315**
**B1**

(12)

# EUROPÄISCHE PATENTSCHRIFT

(45) Veröffentlichungstag der Patentschrift:
23.06.89

(21) Anmeldenummer: 85107311.4

(22) Anmeldetag: 13.06.85

(51) Int. Cl.⁴: **A 61 K 9/20**, A 61 K 9/22, A 61 J 3/10

(54) **Magensaftresistent überzogene zylindrische Pankreatin-Mikrotabletten.**

(30) Priorität: 19.06.84 DE 3422619
19.06.84 DE 8418439 U

(43) Veröffentlichungstag der Anmeldung:
02.01.86 Patentblatt 86/1

(45) Bekanntmachung des Hinweises auf die Patenterteilung:
23.06.89 Patentblatt 89/34

(84) Benannte Vertragsstaaten:
AT BE CH DE FR GB IT LI NL SE

(56) Entgegenhaltungen:
DE-A-2 033 121
FR-A-1 570 421
GB-A-2 039 737

(73) Patentinhaber: BASF Aktiengesellschaft, Carl-Bosch- Strasse 38, D-6700 Ludwigshafen (DE)

(72) Erfinder: Pich, Claus Hinrich, Dr., An der Duene 3, D-2082 Moorrege (DE)
Erfinder: Moest, Thomas, Dr., An der Duene 9, D-2082 Moorrege (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Verabreichung von Medikamenten in Form von Pellets, die z. B. in Kapseln gefüllt sein können, ist der Verabreichung in Form kompakter Tabletten in der Regel vorzuziehen, weil bei dem Multi-Unit-Dose-System Pellets im Gegensatz zum Single-Unit-Dose-System Tablette lokal sehr hohe Wirkstoffkonzentrationen im Magen-Darm-Trakt vermieden werden. Eine gleichmäßige Füllung von Kapseln erfordert aber eine gleichmäßige Größe und Form der Pellets. Außerdem ist die in der Regel angestrebte möglichst gleichmäßige Wirkstofffreisetzung pro Zeiteinheit im Fall von retardierten Pellets nur bei gleichmäßiger Größe und Form der Pellets möglich. Das gleiche gilt für magensaftresistent überzogene Pellets. Nur bei regelmäßig geformten Partikeln erhält man mit geringsten Mengen an Überzugsmitteln einen gleichmäßigen Überzug.

Es ist ein dem Fachmann geläufiges Problem, herkömmliche Pellets durch darmsaftlösliche Überzüge in der Weise magensaftresistent zu formulieren, daß der in den Pellets enthaltene Wirkstoff sicher vor den Einwirkungen des sauren Mediums im Magen geschützt wird. Die Notwendigkeit des Magensäureschutzes besteht insbesondere bei säureempfindlichen Substanzen, z. B. dem Enzym Lipase. Eine magensaftresistente Lackierung erfordert bei derartigen Pellets üblicherweise eine sehr große Lackmenge, die bis zu 50 % des Gesamtgewichtes der lackierten Pellets ausmacht. Trotzdem sind auch derartige magensaftresistente Pellets meistens nur magensaftresistent in dem Sinne, daß der Wirkstoff nicht durch die Lackschicht in die Magensäure diffundiert, nicht jedoch in der zu fordernden Weise, daß die Magensäure nicht umgekehrt durch den Lack in das Pelletinnere hineindiffundiert.

Es ist auch wünschenswert, Pellets mit den genannten Eigenschaften herstellen zu können, die von solcher Gleichmäßigkeit sind, daß eine Einzeldosierung möglich wird. Einzeldosierbarkeit bedeutet, daß jedes einzelne Pelletteilchen den Anforderungen des Europäischen Arzneibuches zu den dort beschriebenen "single-unit-dose"-Formen entsprechen muß. Die notwendige Gewichtseinheitlichkeit ist vorgegeben bei Tabletten im Europäischen Arzneibuch, Band III, S. 77.

Die Vorschriften bezüglich der Zerfallszeiten von nicht überzogenen Tabletten a.a.O. S. 235 bzw. von magensaftlöslichen überzogenen Tabletten a.a.O. S. 237 bzw. von magensaftresistent überzogenen Tabletten a.a.O. Seite 237 müssen von jedem Pelletteilchen eingehalten werden.

Üblicherweise werden Pellets durch Pelletierverfahren hergestellt, z. B. auf dem Pelletierteller, auf Pelletiertrommeln, im Drageekessel oder mit Hilfe anderer Geräte zur aufbauenden Granulierung oder durch Extrudieren, Zerschneidung der Stränge und Abrunden der so erhaltenen zylindrischen Teilchen auf entsprechenden üblichen Geräten. Diese Verfahren sind beispielsweise in R. Voigt, Lehrbuch der pharmazeutischen Technologie, 2. Auflage, VEB Verlag Volk und Gesundheit, Berlin 1975, 158 - 169, sowie in Hagers Handbuch der pharmazeutischen Praxis, 4. Auflage, Springer-Verlag, Berlin - Heidelberg - New York, 1971, Band VII A, 312 - 318 beschrieben.

Alle diese Verfahren haben den Nachteil, daß ein breites Kornband entsteht, so daß Über- und Unterkorn abgetrennt werden müssen, außerdem ist häufig die Form und/oder die Oberflächenstruktur ungleichmäßig. Bei allen diesen Verfahren wird ein Lösungsmittel eingearbeitet und anschließend verdampft, so daß stets eine poröse Struktur entsteht. Die einzelnen Partikeln unterliegen starken Gewichtsschwankungen. Eine Einzeldosierung ist nicht möglich weil die Forderungen des Europäischen Arzneibuches nicht erfüllt werden.

Gemäß Fachleuten aus dem Bereich der Hersteller von Tablettiermaschinen und von Preßwerkzeugen, d.h. von Matrizen und Stempeln, ist es schwierig, kleinere Preßlinge herzustellen. Die Gründe hierfür liegen in der Empfindlichkeit der dünnen Stempel, die beim Einsatz üblicher Maschinen gestaucht werden und abbrechen, in der erforderlichen Präzision der Tablettiermaschinen und in den Anforderungen an die Preßmasse hinsichtlich Rieselfähigkeit, Korngröße und Korngrößenverteilung. Zwar beschreibt das Französische Patent 1 570 421 bereits Tabletten von 1 bis 2 mm Durchmesser, doch ohne jedes Beispiel und ohne jede Erwähnung der genannten Schwierigkeiten. Derartige Mikrotabletten sind auch nie auf dem Markt erschienen, geschweige denn solche mit Pankreatin als Wirkstoff.

In der DE-A-2 033 121 werden "Mikrotabletten" von 6 mm Durchmesser beschrieben. Diese sind für die Zwecke der Erfindung viel zu groß.

Der Erfindung lag die Aufgabe zugrunde, Partikel mit den eingangs geschilderten Eigenschaften herzustellen, die Pankreatin als Wirkstoff enthalten.

Die Lösung dieser Aufgabe besteht in magensaftresistent überzogenen zylindrischen Mikrotabletten mit konvexer Ober- und Unterseite, deren Zylinder Durchmesser und -Höhe unabhängig voneinander im Bereich vom 1,0 bis 2,5 mm liegen, dadurch gekennzeichnet, daß

a) Durchmesser und Höhe sich zueinander wie 1 : (0,5 bis 1,5) verhalten, vorzugsweise im Bereich von 1,5 bis 2,3 mm liegen,

b) der Wölbungsradius r der konvexen Ober- und Unterseiten sich zum Zylinderdurchmesser wie (0,6 bis 1,5) : 1 verhält und

c) die Tablette als Wirkstoff Pankreatin in hoher Konzentration enthält.

Bevorzugt ist das Einzelgewicht im Bereich vom 1 bis 20 mg und die relative Standardabweichung des mittleren Gewichtes kleiner als 4 % (n = 50). Zu ihrer Herstellung verpreßt man eine gut rieselfähige Pankreatin-Preßmasse mit einem maximalen Partikeldurchmesser von 30, vorzugsweise 20 % des Tablettendurchmessers und weniger als 10, vorzugsweise weniger als 5 Gewichtsprozent Staubanteil (mit Partikeldurchmessern unter 50 µm) mit einer Kraft von 0,4 bis 3, vorzugsweise 1 bis 2 kN.

Die erforderlichen Pankreatin-Preßmassen mit der genannten Partikelgröße und dem genannten geringen Staubanteil erhält man zweckmäßig durch Mahlen von größeren Partikeln, wobei Mühlen mit geringer Scherwirkung zu bevorzugen sind. Außerdem erfordert das Verfahren neuartige Tablettiermaschinen, wie sie bisher noch nicht im Handel sind. Sie müssen nicht nur entsprechend kleine Matrizen und Stempel besitzen, sondern es muß auch der Meßbereich für die anzuwendende Preßkraft den geringeren Dimensionen der Mikrotablette angepaßt sein. Die Werkzeuge müssen besonders präzise geführt werden. Eine sensible Steuerung der Dosierung ist erforderlich, um Abweichungen des mittleren Tablettengewichtes während des Preßverlaufes zu vermeiden, da eine Überfüllung der Matrizen zu einer Überlastung der Werkzeuge führt. Schließlich muß für eine sehr gut funktionierende Abstreifvorrichtung gesorgt werden, die die Mikropreßlinge sorgfältig ohne Beschädigung, aber auch zuverlässig ohne Rückstand von der Matrize in die Auslaufvorrichtung führt.

Der Wölbungsradius r der konvexen Ober- und Unterseite der zylindrischen Mikrotabletten liegt im Bereich von 0,6 bis 1,5, vorzugsweise 0,7 bis 0,9-fachen Zylinderdurchmesser. Bei kleineren Wölbungsradien (Kugelform) halten die Werkzeuge den erforderlichen Druck nicht aus, bei größeren nähert man sich flachen Ober- und Unterseiten (Wölbungsradius unendlich) mit dem Nachteil, daß die Kanten beim Umhüllen (Überziehen) stören und für mechanische Beschädigungen anfällig sind.

Mit der Höhe der Tablette ist deren maximale Abmessung auf der Zylinderachse gemeint.

"Gut rieselfähig" soll bedeuten, daß der ctg φ des Böschungswinkels gemäß Bestimmung DIN 53916 größer als 1,2, vorzugsweise größer als 1,4 ist.

Die erfindungsmäßen Mikrotabletten enthalten Pankreatin neben üblichen galenischen Hilfsstoffen.

Das Gewicht der erfindungsgemäßen Mikrotabletten liegt im Bereich von 1 bis 20 mg, vorzugsweise im Bereich von 5 bis 10 mg. Die relativen Standardabweichungen der mittleren Gewichte von 50 (= n; vgl. Anspruch 4) gewogenen Mikrotabletten, die nach diesem Verfahren hergestellt werden, liegen unter 4 %, im allgemeinen sogar unter 2,5 %. Sie entsprechen den Forderungen des Europäischen Arzneibuches bezüglich der Gewichtseinheitlichkeit von Tabletten. Zur Definition der Standardabweichung vgl. Lehrbücher der Statistik, z. B. Siegfried Noack, Auswertung von Meß- und Versuchsdaten mit Taschenrechner und Tischcomputer, Walter de Gruyter Verlag, Berlin, New York, 1980, S. 192 - 201.

Konventionelle Pellets mit unregelmäßiger Größe und Form ergeben nach einer Retardierung durch einen retardierenden Lacküberzug individuell stark streuende Freisetzungscharakteristiken des Wirkstoffs. Dies ist zurückführbar auf die unterschiedliche Größe der Oberflächen von Pellets mit unterschiedlichen Durchmessern.

Kleinere Pellets mit großer massebezogener Oberfläche benötigen mehr Lack für eine gleich dicke und damit gleich wirksame Lackschicht als größere Pellets mit kleinerer massebezogener Oberfläche. Verschärft wird diese Breitbandigkeit der zeitlichen Freisetzungsverläufe durch die Einflüsse von Formfaktoren, denn Partikeln mit Kanten und Ecken oder erhabenen Oberflächenstrukturen benötigen mehr Lack, um diese Unebenheiten zu überdecken.

Mit dem Auftragen einer mittleren Lackmenge beim Überziehen werden nur wenige Pellets den gewünschten mittleren Freisetzungsverlauf erreichen, große und ebenförmige Partikeln werden langsamer freisetzen, kleine und "unebene" Partikeln werden schneller freisetzen.

Es gelingt ohne besonderen Aufwand, die erfindungsgemäßen Mikrotabletten vollständig magensaftresistent herzustellen. Man kann sie anstelle herkömmlicher Pellets in Kapseln abfüllen. Mit Hilfe der bereits genannten Überzugsverfahren können homogen magensaftresistente Pellets durch Auftragen von Lackierungen auf Basis bekannter Lacksysteme wie Celluloseacetatphthalat oder Hydroxypropylmethylcellulosephthalat erzielt werden. Der Lackverbrauch ist dabei, je nach Pelletgröße, nicht größer als 25 % G/G; in vielen Fällen genügen 10 % G/G.

Überraschenderweise sind Pankreatin-Mikrotabletten mit einem Durchmesser von weniger als 2,5 mm nicht nur überhaupt preßbar, sondern sie weisen zudem unerwartet gute Preßeigenschaften auf. Die Preßbarkeit von Pankreatin-Preßmassen wird erstaunlicherweise besser bei solch kleinen Preßlingen. Pankreatin läßt sich mit einem Gehalt von 99,5 % verpressen.

Werden Massen mit so hohem Pankreatingehalt mit herkömmlichen runden Preßwerkzeugen mit 10 mm Durchmesser verpreßt, so entstehen Tabletten von so ungenügender Festigkeit, daß sie nicht weiterverarbeitet werden können.

Die im Beispiel genannten Teile und Prozente beziehen sich auf das Gewicht.

**Beispiel**

Pankreatin, das nach dem Extraktionsverfahren hergestellt wurde, wurde mit einer Walzenmühle so zerkleinert, daß der Anteil über 0,5 mm bei 0,8 % und der Staubanteil unter 50 µm bei 3,5 % lag.

Nach Mischen von 1 990 g dieses Pankreatins mit 10 g Magnesiumstearat in einem 5 l-Labormischer wies die Mischung eine Rieselfähigkeit gemäß DIN 53916 mit ctg φ = 1,33 auf.

Diese Preßmasse wurde auf einer instrumentierten Exzenterpresse mit präziser Stempelführung zu Mikrotabletten im Format von 2,25 mm Durchmesser und 2,2 mm Höhe mit einer Preßkraft von 2 kN verpreßt. Der Wölbungsradius betrug 1,7 mm. Das Durchschnittsgewicht von 50 Mikrotabletten lag bei 8,5 mg, die relative Standardabweichung bei 2,4 %.

Die Mikrotabletten entsprachen den Forderungen des Arzneibuches für Gewichtseinheitlichkeit bei Tabletten.

Die Pankreatin-Mikrotabletten wurden in einer rotierenden Lochtrommel (Accela Cota 24" der Fa. Manesty, Liverpool mit 0,3 mm laserstrahlgelochter Trommel) mit einer Lösung von Hydroxypropylmethylcellulosephthalat in Isopropanol-Methylenchlorid 3 : 7 mit Hilfe einer Zweistoffdüse überzogen. Die Konzentration der Lösung betrug 7 % G/G. Die Gesamtmenge des Hüllpolymeren betrug 14 % G/G, bezogen auf die überzogenen Pankreatin-Mikrotabletten. Der Polymerenlösung wurden als Weichmacher 20 % G/G Dibutylphthalat, bezogen auf das Polymerenmaterial, zugesetzt.

Die Lackierung wurde so gesteuert, daß bei einer Dosierung der Lacklösung von 40 ml/min die Produkttemperatur im Bereich von 24 - 26°C verweilte.

Die magensaftresistenten Pankreatin-Mikrotabletten konnten sehr leicht und genau mit üblichen Geräten in Hartgelatine-Steckkapseln abgefüllt werden.

Die Magensaftresistenz wurde nach der Vorschrift der Ph. Eur. geprüft. Darüber hinaus wurde das Eindringen von künstlicher Magensäure in die Pellets dadurch bestimmt, daß nach 2-stündigem Einwirken der Säure der Gehalt an Lipase bestimmt und mit dem Ausgangswert verglichen wurde.

Bei den erfindungsgemäß hergestellten magensaftresistenten Mikrotabletten konnte kein Abfall der Lipase-Aktivität festgestellt werden.

Vergleichsweise wurde ein Handelsprodukt mit magensaftresistenten Pellets untersucht. Dieses Produkt ist zwar magensaftresistent im Sinne der Arzneibuchvorschrift, jedoch wurde nach 2-stündigem Einwirken von künstlicher Magensäure ein Abfall der Lipase-Aktivität um 60 % ermittelt. Eine Bestimmung der Lackmenge bei diesem Produkt ergab den Wert von 38 % G/G.

Zum Vergleich des Tablettierverhaltens wurden mit der gleichen 99,5 %-igen Pankreatin-Preßmasse runde 10 mm Tabletten hergestellt.

Diese haben nur eine geringe Bruchfestigkeit und weisen hohen Abrieb auf. Ein Lackierversuch in der Wurster-Anlage mußte abgebrochen werden, da Bruch- und Abriebspartikel keine sinnvolle Lackierung zuließen.

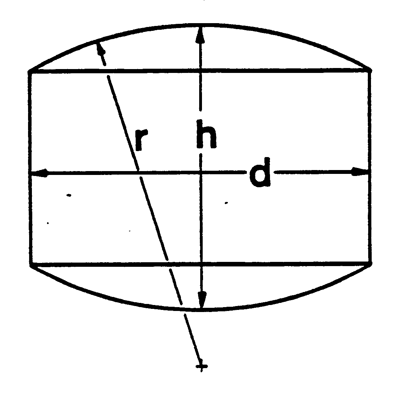

**Patentansprüche**

1. Magensaftresistent überzogene zylindrische Mikrotablette mit konvexer Ober- und Unterseite, deren Zylinder Durchmesser und -Höhe unabhängig voneinander im Bereich von 1,0 bis 2,5 mm liegen, dadurch gekennzeichnet, daß

a) Durchmesser und Höhe sich zueinander wie 1 : (0,5 bis 1,5) verhalten,

b) der Wölbungsradius r der konvexen Ober- und Unterseiten sich zum Zylinderdurchmesser wie (0,6 bis 1,5) : 1 verhält und

c) die Tablette als Wirkstoff Pankreatin in hoher Konzentration enthält.

2. Zylindrische Mikrotabletten gemäß Anspruch 1, dadurch gekennzeichnet, daß Durchmesser und Höhe im Bereich von 1,5 bis 2,3 mm liegen.

3. Zylindrische Mikrotabletten gemäß Anspruch 1 oder 2, dadurch gekennzeichnet, daß das Einzelgewicht im Bereich von 1 bis 20 mg liegt und die relative Standardabweichung des mittleren Gewichtes kleiner als 4 % (n = 50) ist.

**Claims**

1. A cylindrical microtablet which has a coating resistant to gastric juice and possesses convex upper and lower faces and whose cylinder diameter and cylinder height independently of one another are from 1.0 to 2.5 mm, wherein

a) the ratio of diameter to height is 1 : (0.5 to 1.5),

b) the ratio of the radius of curvature r of the convex upper and lower faces to the cylinder diameter is (0.6 to 1.5) : 1 and

c) the tablet contains pancreatin in high concentration as an active compound.

2. A cylindrical microtablet as claimed in claim 1, wherein the diameter and height are from 1.5 to 2.3 mm.

3. A cylindrical microtablet as claimed in claim 1 or 2, wherein the individual weight is from 1 to 20 mg and the relative standard deviation of the mean weight is less than 4 % (n = 50).

**Revendications**

1. Microtablette cylindrique enduite, résistante au suc gastrique à faces inférieure et supérieur, convexes, dont le diamètre de cylindre et la hauteur sont situées indépendamment l'une de l'autre, dans le domaine de 1,0 à 2,5 mm caractérisée par le fait que

a) diamètre et hauteur sont, entre eux, dans le rapport de 1/(0,5 à 1,5)

b) le rayon de courbure r des faces supérieure et inférieure convexes est, avec le diamètre de cylindre, dans le rapport de (0,6 à 1,5)/1 et

c) la tablette contient, à haute concentration, de la pancréatine, comme principe actif.

2. Microtablette cylindrique selon la revendication 1, caractérisée par le fait que diamètre et hauteur se situent dans le domaine de 1,5 à 2,3 mm.

3. Microtablette cylindrique selon la revendication 1 ou 2, caractérisée par le fait que le poids individuel est situé dans le domaine de 1 à 20 mg et l'écart-type relatif du poids moyen est inférieur à 4 % (n = 50).